(19) Europäisches Patentamt
European Patent Office
Office européen des brevets

(11) EP 3 587 389 A1

(12) EUROPEAN PATENT APPLICATION

(43) Date of publication:
**01.01.2020 Bulletin 2020/01**

(21) Application number: **18000565.4**

(22) Date of filing: **28.06.2018**

(51) Int Cl.:
***C07C 67/343*** (2006.01) ***C07C 69/732*** (2006.01)
***C07C 45/72*** (2006.01) ***C07C 49/248*** (2006.01)
***A61K 8/35*** (2006.01) ***A61K 8/37*** (2006.01)
***A61K 31/12*** (2006.01) ***A61K 31/216*** (2006.01)
***A61Q 17/04*** (2006.01) ***A61P 17/18*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **INSTYTUT FARMACEUTYCZNY**
**01-793 Warszawa (PL)**
- **Uniwersytet Wroclawski**
**50-137 Wroclaw (PL)**

(72) Inventors:
- **Sidoryk, Katarzyna**
**01-850 Warszawa (PL)**
- **Cybulski, Marcin**
**03-254 Warszawa (PL)**
- **Jaromin, Anna**
**51-253 Warszawa (PL)**
- **Filipczak, Nina**
**51-163 Wroclaw (PL)**

(54) **PROCESS FOR THE PREPARATION OF CAFFEIC ACID DERIVATIVES**

(57) A process for the preparation of caffeic acid derivatives which are substituted on the aromatic ring with at least two hydroxyl groups by reaction of aromatic aldehydes with unprotected hydroxyl groups and ylides under Horner-Wadsworth-Emmons reaction conditions, wherein the reaction is carried out in water.

EP 3 587 389 A1

## Description

### Field of the invention

The present invention relates to the process for the preparation of the polyphenolic caffeic acid derivatives *via* a modified Wittig reaction. The invention also pertains to the use of the polyphenolic caffeic acid derivatives as the antioxidants, especially in cosmetics.

### Background of the invention

Caffeic acid (CA) is a natural polyphenolic acid which is synthesised by plants as a secondary metabolite. Caffeic acid as well as its natural and synthetic derivatives show a potent antioxidative activity, even at low concentrations. Moreover, it has been proved that the caffeic acid and its analogues show anti-inflammatory, antibacterial, anti-viral and anti-tumor activities [P. Zhang et al., Bioactivity and chemical synthesis of caffeic acid phenethyl ester and its derivatives; Molecules, 19, (2014), pp. 16458-16476; S. Quideau et al., Plant polyphenols: chemical properties, biological activities, and synthesis. Angew. Chem. Int Ed.,50, (2011), pp. 586-621; C.A. Gomes et al. Anticancer activity of phenolic acids of natural or synthetic origin: a structure-activity study. J. Med. Chem., 46, (2003), pp 5395-5401]. Recent investigations have proved that caffeate esters, especially methyl caffeate, are sucrase and maltase inhibitors [H.M. Eid et al., Structural constraints and the importance of lipophilicity for the mitochondrial uncoupling activity of naturally occurring caffeic acid esters with potential for the treatment of insulin resistance. Biochem. Pharmacol., 79, (2010), pp. 444-454; K. Takahashi et al., Methyl caffeate as an α-glucosidase inhibitor from Solanum torvum fruits and the activity of related compounds. Biosci. Biotechnol. Biochem., 74 (4), (2010), pp. 741-745; J. Ganugapati et al. Molecular docking studies of antidiabetic activity of cinnamon compounds. Asian J. of Pharm. and Clinical Res., 7,2 (2014)].

Various biological studies on polyphenolic acid molecules have evidenced that their varied biological activities stem from their anti-oxidative properties [S. M. Fiuza et al., Phenolic acid derivatives with potential anticancer properties - a structure-activity relationship study. Part 1: Methyl, propyl and octyl esters of caffeic and gallic acids. Bioorg. Med. Chem., 12, (2004), pp. 3581-3589; L.S. Monteiro et al., Synthesis and preliminary biological evaluation of new phenolic and catecholic dehydroamino acid derivatives. Tetrahedron., 73, (2017), pp. 6199-6209]. The antioxidative activity of polyphenolic acids results from their acclaimed capability to scavenge reactive oxygen species (ROS) which include radical and non-radical oxygen species such as $O_2^-$, HO, NO, $H_2O_2$ [J.H. Chen, H. Chi-Tang, Antioxidant activities of caffeic acid and its related hydroxycinnamic acid compounds. J. Agric. Food Chem., 7 (45), (1997), pp. 2374-2378; Y. Sato et al., In vitro and in vivo antioxidant properties of chlorogenic acid and caffeic acid. Int J. Pharm., 1-2 (403), (2010), pp. 136-138; S. Losada-Barreiro, C. Bravo-Dfaz, Free radicals and polyphenols: The redox chemistry of neurodegenerative diseases. Eur. J. Med. Chem., 133, (2017), pp. 379-402]. The latest studies showed that the presence of free hydroxyl groups, their number and position in the phenyl ring are crucial for the strength of the antioxidative activity, while their protection renders them inactive [V. Srivastava et al., Synthesis of diverse analogues of Oenostacin and their antibacterial activities. Bioorg. Med. Chem., 15, (2007), pp. 518-525]. Furthermore, the type of a spacer between the carboxylic group and the aromatic ring of polyphenolic acids also markedly influences their antioxidant profile - derivatives with methylenic, ethylenic and unsaturated chains have the highest activity [Ch. Siquet et al., Antioxidant profile of dihydroxy- and trihydroxyphenolic acids - A structure-activity relationship study. Free Radical Res., 40(4), (2006), pp. 433-442; P.C. Kuo et al., Isolation of a natural antioxidant, dehydrozingerone from Zingiber officinale and synthesis of its analogues for recognition of effective antioxidant and antityrosinase agents. Arch. Pharm. Res., 28, 5, (2005), pp. 518-528].

Due to these advantageous biological effects, polyphenolic acids and their derivatives have currently become the essential instrument for the prevention or treatment of many diseases, and therefore they have found wide application in the cosmetic and pharmaceutical field [I.E. Alvarado et al., Fungal biotransformation of p-coumaric acid into caffeic acid by Pycnoporus cinnabarinus: an alternative for producing a strong natural oxidant. World J., Microbiol. Biotechnol., 19, (2003), pp. 157-160; P.A Kroon, G. Williamson, Hydroxycinnamates in plants and food: current and future perspectives. J. Sci. Food Agric., 79, (1999), pp. 355-361]. Phenolic acids, and in particular caffeic acid, are also important scaffolds for the synthesis of a variety of biologically active compounds [M. Touaibia et al., Caffeic acid, a versatile pharmacophore: an overview. Mini Rev. Med. Chem., 11, (2011), pp. 695-713].

Most common commercially available polyphenolic acids are usually obtained by extraction from their natural plant sources, as well as in the biotechnological process [T. Furuya et al., Biotechnological production of caffeic acid by bacterial cytochrome P450 CYP199A2. Appl. Environ Microbiol., 78(17), (2012), pp. 6087-6094]. However, chemical synthesis also plays an important role in accessing pure polyphenolic acids. In general, there are two most important routes to obtain α,β-unsaturated systems. The first approach uses a modified Wittig reaction, namely the Horner-Wadsworth-Emmons (HWE) reaction [A. Jafari, M. Ghadami, Efficient synthesis of α,β-unsaturated ketones with trans-selective Horner-Wadsworth-Emmons reaction in water. Environ. Chem. Lett., 14, (2016), pp. 223-228]. The second

exploits Knoevenagel [B. List. Angew. Chem. Int. Ed. (2010), 49, 1730-1734], and aldol condensations [G. Guillena, C. Najera, D. Ramon. Enantioselective direct aldol reaction: the blossoming of modern organocatalysis. Tetrahedron: Asymmetry (2007), 18, 2249-2293.]. Unfortunately, the straightforward application of these known methods to obtain different analogues of the caffeic acid has some drawbacks, such as the multiplicity of procedures, side reactions, high temperature, long reaction time, low yield and low stereoselectivity [K. Takahashi et al., Methyl caffeate as an α-glucosidase inhibitor from Solanum torvum fruits and the activity of related compounds. Biosci. Biotechnol. Biochem., 74 (4), (2010), pp. 741-745; N. Saito et al., Guanidine-catalyzed asymmetric synthesis of 2,2-disubstituted chromane skeletons by intramolecular oxa-Michael addition. Eur. J. Org. Chem., (2008), pp. 2759-2766; V. Srivastava et al., Synthesis of diverse analogues of Oenostacin and their antibacterial activities. Bioorg. Med. Chem. 15, (2007), pp. 518-525].

The attempts of the present Inventors to apply the Knoevenagel reaction to obtain the derivatives of the caffeic acid substituted with three phenyl groups have failed. The reaction of the unprotected 2,3,4-trihydroxybenzaldehyde with malonic acid in pyridine, in the presence of piperidine, has led to the decomposition of the substrates. The protection of hydroxyls with benzyl- or silyl-protecting groups has made the preparation of the desired product by Knoevenagel and Wittig reactions impossible, probably due to the presence of the large protecting groups in the aromatic aldehyde.

To improve the efficiency of the Horner-Wadsworth-Emmons reaction, a number of variations on the reaction conditions have been reported. These optimizations included, e.g., increasing the temperature, pressure, presence of additives, irradiation with microwaves or light, use of silica gel or ionic solvents [V.J. Patil, U. Mavers. Wittig reactions in the presence of Silica gel. Tetrahedron Lett, 37, (1996), pp. 1281-1284; N.S. Isaacs, G.N. El-Din. The application of high pressure to some difficult Wittig reactions. Tetrahedron Lett., 28, (1987), pp. 2191-2192; D.L. Hooper, S. Garagan, M.M. Kayser. Lithium cation-catalyzed Wittig reactions. J. Org. Chem., 59, (1994), 1126-1128; V.L. Boulaire, R. Gree. Wittig reactions in the ionic solvent [bmim][BF4]. Chem. Commun., (2000), pp. 2195-2196; A. Spinella et al., Microwave accelerated Wittig reactions of stabilized phosphorus ylides with ketones under solvent-free conditions. Synlett, (1997), pp. 93-94]. Latest investigation demonstrated that a modified Wittig reaction could be carried out in water which appeared to be an efficient medium, particularly for the aromatic aldehydes. Although the starting material and products were very slightly soluble in water, the rate of reactions was fast Moreover, the high yields 80-98%, short olefination time, and very high E/Z-isomeric ratios in the olefination products prompted a detailed study of water as the essential medium in modified Wittig reactions [A.A. Jafari, M. Ghadami. Efficient synthesis of α,β-unsaturated ketones with trans-selective Horner-Wadsworth-Emmons reaction in water. Environ. Chem. Lett., 14, (2016), pp. 223-228; J. Dambacher et al., Water is an efficient medium for Wittig reactions employing stabilized ylides and aldehydes. Tetrahedron Lett, 46 (2005), pp. 4473-4477; A. El-Batta et al., Wittig reactions in water media employing stabilized ylides with aldehydes. Synthesis of α,β-unsaturated esters from mixing aldehydes, α-bromoesters, and Ph3P in aqueous NaHCO3. J. Org. Chem., 72, (2007), pp. 5244-5259]. Bergdahl et al. demonstrated in only one of their experiments that the water medium can be very effective for the p-hydroxybenzaldehyde substrate, having an unprotected hydroxyl group [J. Dambacher et al., Water is an efficient medium for Wittig reactions employing stabilized ylides and aldehydes. Tetrahedron Lett., 46 (2005), pp. 4473-4477; A. El-Batta et al., Wittig reactions in water media employing stabilized ylides with aldehydes. Synthesis of α,β-unsaturated esters from mixing aldehydes, α-bromoesters, and Ph3P in aqueous NaHCO3. J. Org. Chem., 72, (2007), pp. 5244-5259]. The appropriate cinnamate product was obtained with a 92% yield, contrary to the same procedure in refluxing DCM which provided the same compound with only an 8% yield [A. Nonnenmacher et al., Application of highpressure on Wittig reactions with resonance stabilized ylides. Liebigs Ann. Chem., 12, (1983), pp. 2135-2140].

Despite unquestionable advantages of carrying out the Horner-Wadsworth-Emmons reaction in water, there are no literature reports on its suitability in relation to aromatic aldehydes containing more than one unprotected hydroxyl group.

Thus, it was the aim of the invention to develop such Horner-Wadsworth-Emmons reaction conditions that would be efficient for the synthesis of the caffeic acid derivatives containing additional hydroxyl groups (ie. compounds with two or three hydroxyl groups in the aromatic ring) and/or starting from the aromatic aldehydes with the hydroxyl groups in the diverse positions of aromatic ring.

## Summary of the invention

In one aspect, the invention provides the process for the preparation of the caffeic acid derivatives substituted with at least two hydroxyl groups in the aromatic ring, and represented by formula (I)

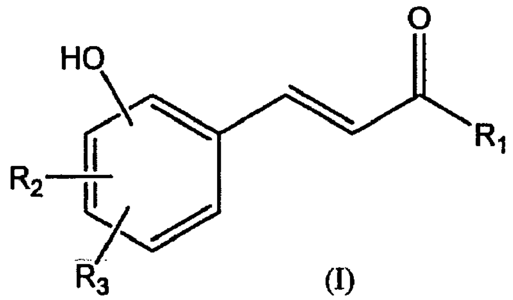

(I)

wherein

$R_1$ is $-CH_3$ or $-OCH_3$,

$R_2$ is OH or $-OCH_3$, and

$R_3$ is H or OH,

in the reaction of an aromatic aldehyde with the unprotected hydroxyl groups represented by formula (II)

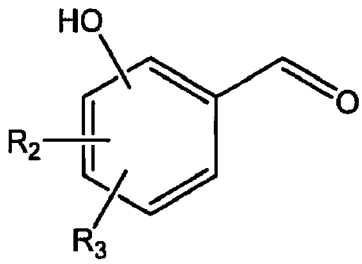

(II)

and ylide of formula (III)

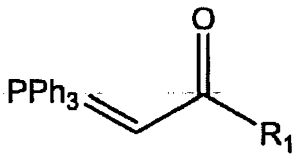

(III)

wherein $R_1$, $R_2$ and $R_3$ have the meaning defined for formula (I),
under the Horner-Wadsworth-Emmons reaction conditions, wherein the reaction is carried out in water.

In one embodiment of the invention, the process may apply to the preparation of the compounds of formula (I) substituted with two OH groups, wherein $R_1$ is $-OCH_3$ or $-CH_3$, OH is in the position 2 of the aromatic ring, $R_2$ is OH, and $R_3$ is H.

In another embodiment of the invention, the process may apply to the preparation of the compounds of formula (I) substituted with two OH groups, wherein $R_1$ is - $OCH_3$ or $-CH_3$, $R_2$ is $-OCH_3$ in the position 2, and $R_3$ is OH in the position 3 of the aromatic ring.

In yet another embodiment of the invention, the process may apply to the preparation of the compounds of formula (I) substituted with three OH groups, wherein $R_1$ is $-OCH_3$ or $-CH_3$, and both $R_2$ and $R_3$ are OH, under the provision that when $R_1$ is $-OCH_3$, neither $R_2$ nor $R_3$ is in the position 5 of the aromatic ring.

## Detailed description of the invention

The suspension of an appropriate aromatic aldehyde (**II**) (1 eq.) and ylide (**III**) (1.3 - 1.5 eq) is stirred in water at 70-100°C, preferably at 90°C, for 0.5-24 h. Then, the heterogeneous reaction mixture is cooled to room temperature, and the aqueous phase is extracted with an organic solvent, eg. dichloromethane. The solvent is evaporated under reduced pressure. Column chromatography of the residue gives the E-alkene as the major product

The Horner-Wadsworth-Emmons reactions according to the invention, ie. starting from the aromatic aldehydes (II) substituted with two or three hydroxyl groups, were compared with the same reaction starting from the aldehydes with one hydroxyl group in the diverse positions of aromatic ring with the respect to the phenol group as the comparative examples.

The reaction times of the Horner-Wadsworth-Emmons reaction and yields of the resulting products are presented in Table 1 below.

Table 1.

| No. | Aldehyde (II) | Ylide (III) | Product (E isomer) | Time (h) | Yield (%) |
|---|---|---|---|---|---|
| 1.* | 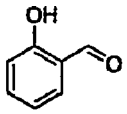 | 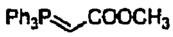 | 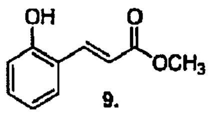 9. | 0.5 | 82 |
| 2.* | 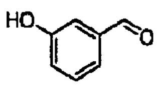 | 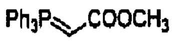 | 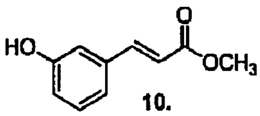 10. | 1.0 | 86 |
| 3.* | 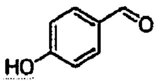 | 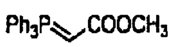 | 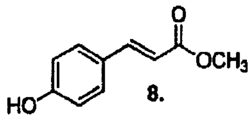 8. | 1.0 | 91 |
| 4. | 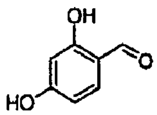 | 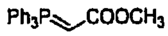 | 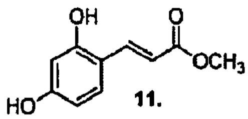 11. | 5.0 | 86 |
| 5. | 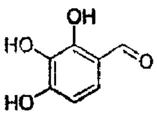 | 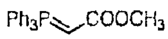 | 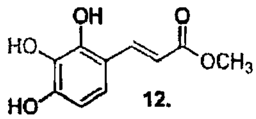 12. | 1.0 | 74 |
| 6. | 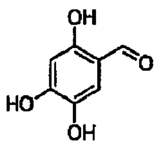 | 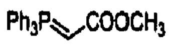 | 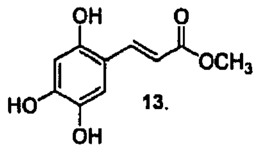 13. | 1.0 | 37 |
| 7. | 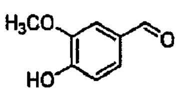 | 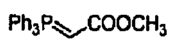 | 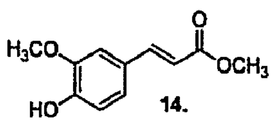 14. | 0.5 | 99 |
| 8.* | 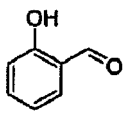 | 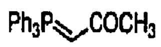 | 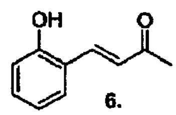 6. | 2.5 | 85 |
| 9.* | 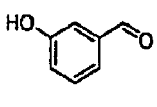 | 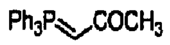 | 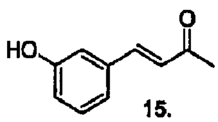 15. | 3.0 | 97 |

(continued)

| No. | Aldehyde (II) | Ylide (III) | Product (E isomer) | Time (h) | Yield (%) |
|---|---|---|---|---|---|
| 10.* | 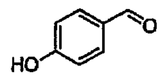 | 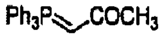 | 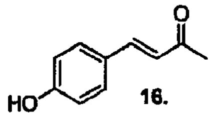 16. | 2.0 | 83 |
| 11. | 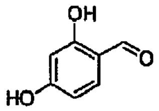 | 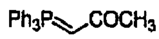 | 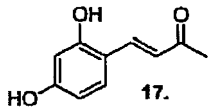 17. | 2.0 | 78 |
| 12. | 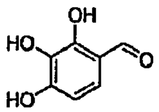 | 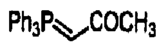 | 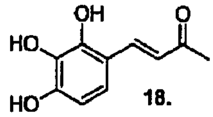 18. | 0.5 | 70 |
| 13.** | 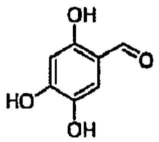 | 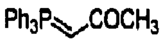 | 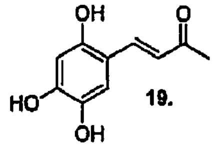 19. | 5.0 | 0 |
| 14. | 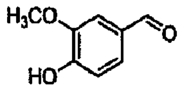 | 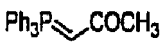 | 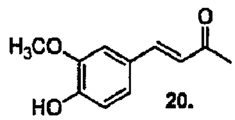 20. | 0.5 | 98 |
| * *Comparative compound*<br>** *Compound not obtained* | | | | | |

Although all the starting materials and reaction products are poorly soluble in water, the yields of the reactions are generally high. The presence of only one hydroxyl unprotected group in the substrate's aromatic ring results in the conversions ranging from 82 to 97% (reference examples 1, 2, 3, 8, 9, 10, and examples 7, 14 of the invention). However, for the $Ph_3PCHCOCH_3$ ylide substrate a longer time is required to complete the reaction (reference examples 8, 9, 10).

The reaction of aromatic aldehydes with two unprotected hydroxyl groups in the aromatic ring results in favourable yields. Compounds **11** and **17** were obtained with the yields of 86% and 78%, respectively. In contrast, the modified Wittig protocol worked very well for 4'-hydroxy-3-metoxybenzaldehyde (examples of the invention 7, 14). The reactions with both ylides in water for 30 min at 90°C led to compounds **14** and **20** with 99% and 98% yields, respectively.

The introduction of a third hydroxyl group to the aromatic ring led to poorer results (examples of the invention 5, 6, 12, and 13). Compound **12** was obtained with a moderate 74% yield in 1 hour at 90°C, while the prolonged reaction time caused the decomposition of the substrates. Another cinnamate product **13** was obtained only with a 37% yield due to the difficulty with the isolation and purification of 13 from a much more impure crude material. The reaction of 2,4,5-trihydroxybenzaldehyde with the $Ph_3PCHCOCH_3$ ylide in water did not give product **19,** even though the reaction time was extended to 5 hours. Generally, a greater number of hydroxyl electron donating groups in the benzaldehyde aromatic ring decreased the effectiveness of the conversion. This is consistent with the reported findings stating that electron-donating groups reduce the rate of the Wittig reaction in MeOH [J. Dambacher et al. Water is an efficient medium for Wittig reactions employing stabilized ylides and aldehydes. Tetrahedron Lett, 46 (2005), pp. 4473-4477; A. El-Batta et al., Wittig reactions in water media employing stabilized ylides with aldehydes. Synthesis of $\alpha,\beta$-unsaturated esters from mixing aldehydes, $\alpha$-bromoesters, and Ph3P in aqueous NaHCO3. J. Org. Chem., 72, (2007), pp. 5244-5259].

Thus, water is an effective medium for the Horner-Wadsworth-Emmons reaction. A one-step procedure provides *E*-products with high selectivity and good yields and its application in the synthesis of different polyphenolic compounds turns out to be easy, fast, and environment friendly.

The synthetic method of the preparation of the polyphenolic caffeic acid derivatives in water is advantageous because of mild reaction conditions and the ease of manipulation. The most important advantage of that procedure, in comparison to the prior art methods with a protected hydroxyl group, is the elimination of environmentally unsafe reagents (e.g. $PBr_3$) and harsh reaction conditions.

Moreover, development of an efficient Horner-Wadsworth-Emmons process broadens the spectrum of the

synthetic polyphenolic compounds available for the potential use in supplementation of, among others, pharmaceuticals and cosmetics. Some of the caffeic acid derivatives obtainable due to the process of the invention are known in the art However, some of the caffeic acid derivatives obtainable due to the process of the invention have not been disclosed in the art.

Thus, the second aspect of the invention relates to the new caffeic acid derivatives selected from the group comprising:

3-(2',4',5'-trihydroxyphenyl)-(*E*)-propenoic acid methyl ester, and
4-(2',3',4'-trihydroxyphenyl)-3(*E*')-buten-2-One.

The antioxidative effect of the caffeic acid and its derivatives was tested against lipid peroxidation in the o/w emulsion model and is presented in Table 2 in the Examples section. The high antioxidative activity of the compounds **12,13** and **18,** manifested by the highest protection against the induced oxidation results from the presence of three hydroxyl groups. These results are generally in agreement with radical scavenging activities determined using a DPPH assay. The DPPH scavenging assay is based on the measurement of discolouration resulting from the reduction of a DPPH free radical by an antioxidant Thus, lower $IC_{50}$ values for compounds **12** and **18** from the DPPH assay correspond to high antioxidative activities. However, it seems that the presence of one -OH group is not sufficient to exert such an activity and compounds **9, 10, 8, 6, 15** and **16** have no scavenging abilities.

Having proven the potent antioxidative activity of the synthetized compounds and in the light of encouraging reports concerning significant photo-protection to the skin against the UV-induced erythema (A. Saija et al., Ferulic and caffeic acids as potential protective agents against photooxidative skin damage, J. Sci. Food Agric., 1999, 79, 476-480), reduction of UV-induced skin aging (Saija, A. et al., In vitro and in vivo evaluation of caffeic and ferulic acids as topical photoprotective agents. Int. J. Pharm. 2000, 199, 39-47) as well as the anti-wrinkling activity (Pluemsamran, T. et al., Caffeic acid and ferulic acid inhibit UVA-induced matrix metalloproteinase-1 through regulation of the antioxidant defense system in keratinocyte HaCaT cells. Photochem. Photobiol. 2012, 88, 961-968), preliminary cytotoxicity against skin cells was determined.

The biocompatibility of the synthesized compounds on a normal human dermal fibroblast (NHDF) cell line was investigated by an MTT assay. Fig.1 shows the viability of NHDF incubated with the compounds at the concentrations of 12.5-250 μM. All compounds did not affect the viability of the human cells *in vitro* for 24 h and 48 h, except compounds **13** and **20** when were used at the highest concentration (250 μM).

The caffeic acid derivatives prepared by the process of the invention possess an antioxidative activity and are safe in contact with the skin. Thus, they might be used in cosmetics as the active agents for skin or hair care products.

Another aspect of the invention relates to the use of the caffeic acid derivatives prepared by the process of the invention as antioxidants in cosmetics.

The preferred embodiment of that aspect the invention relates to the use of the caffeic acid derivatives prepared by the process of the invention as the active agents for skin or hair care products.

## Examples

### General materials and methods

Melting points (m.p.) were determined using a Mettler Toledo MP90 apparatus and were uncorrected.

The $^1H$ and $^{13}C$ NMR spectra of all compounds studied were measured in $CDCl_3$ or $CD_3OD$ using Varian-NMR-vnmrs500 and Varian-NMR-vnmrs600 spectrometers at the temperature of 298 K. Standard experimental conditions and standard Varian programs were used. The $^1H$ and $^{13}C$ NMR chemical shifts relate to the TMS. The concentration of all solutions used for the measurements was about 10-20 mg of the compounds in 0.6-0.8 $cm^3$ of the solvent.

The ESI-MS spectra were recorded on a PE Biosystems Mariner mass spectrometer. The progress of the reaction was monitored by thin layer chromatography (TLC) with Merck DC-Alufolien Kieselgel 60 $F_{254}$.

The chemicals and solvents were purchased from Fluka Company. Column chromatography was performed on Merck silica gel 60 (230-400 mesh).

### General synthetic procedure

A suspension of an appropriate aromatic aldehyde **II** (1 eq.), and ylide **III** (1.3 -1.5 eq.) in water (4 -10 mL) was stirred at 90°C for 0.5-24 h. Then, the heterogeneous reaction mixture was cooled to room temperature, and the aqueous phase was extracted with DCM (3 x 10 mL). The solvent was evaporated under reduced pressure. Column chromatography (hexane - ethyl acetate, or chloroform - methanol) of the residue gave the E-alkene as the major product.

**Example 1 (comparative)**

**3-(2'-Hydroxyphenyl)-(*E*)-propenoic acid methyl ester (9)**

2-Hydroxybenzaldehyde (0.82 mmol, 85 μL) was reacted with (methoxycarbonylmethyl)triphenylphosphine (1.23 mmol, 410 mg) in water (4.0 mL) at 90°C for 0.5 h. The crude product was purified using flash chromatography (EtOAc/ hexane, 10:1 → 3:1) to give 82% (120 mg) of 9 as a white solid. m.p. 118-119°C, (reference m.p.: 119-120°C [Chem. Med. Chem. 5, (2010), pp. 598-607]); $^1$H NMR (500 MHz, $CDCl_3$) δ 8.08 (d, 1H, $CH^{\beta}$=$CH^{\alpha}$, J = 16 Hz), 7.46 (dd, aromatic, 1H, J = 1.5 Hz, J = 7.7 Hz), 7.25-7.22 (m, aromatic, 1H), 6.92-6.89 (m, aromatic, 2H), 6.86 (dd, aromatic, 1H, J = 0.73 Hz, J = 8 Hz), 6.64 (d, 1H, CHβ=$CH^{\alpha}$, J = 16 Hz), 3.8 (s, $CH_3$, 3H); $^{13}$C NMR (125 MHz, CDCl3) δ 169.0, 155.5, 141.1, 131.5, 129.2, 121.6, 120.6, 117.8,116.4,51.8; HR-MS (ES-) calc. for $C_{10}H_9O_3$ $(M)^-$: 177.0552. Found: 177.0551.

**Example 2 (comparative)**

**3-(3'-Hydroxyphenyl)-(*E*)-propenoic acid methyl ester (10)**

10 was obtained according to the procedure described in Example 1, using 3-hydroxybenzaldehyde (0.82 mmol, 100 mg) and (methoxycarbonylmethyl)triphenylphosphine (1.23 mmol, 410 mg) in water (4.0 mL) at 90°C for 1 h. The crude product was purified using flash chromatography ( EtOAc/ hexane, 10:1 → 3:1) to give 86% (126 mg) of 10 as a white solid. m.p. 78-81°C, (reference m.p.: 79-81°C [Chem. Med. Chem. 5, (2010), pp. 598-607]); $^1$H NMR (500 MHz, $CDCl_3$) δ 7.66 (d, 1H, $CH^{\beta}$=$CH^{\alpha}$, J = 16 Hz), 7.23 (d, aromatic, 1H, J = 7.9 Hz), 7.09-7.07 (m, aromatic, 1H), 7.03-7.02 (m, aromatic, 1H), 6.86 (dd, aromatic, 1H, J = 0.86 Hz, J = 9 Hz), 6.42 (d, 1H, $CH^{\beta}$=$CH^{\alpha}$, J = 16 Hz), 3.8 (s, $CH_3$, 3H);$^{13}$C NMR (125 MHz, $CDCl_3$) δ 167.9, 156.2,145.0, 135.7, 130.1, 120.7, 117.8, 117.6, 114.5, 51.9; HR-MS (ES-) calc. for $C_{10}H_9O_3$ $(M)^-$: 177.0552. Found: 177.0555.

**Example 3 (comparative)**

**3-(4'-Hydroxyphenyl)-(*E*)-propenoic acid methyl ester (8)**

**8** was obtained according to the procedure described in Example 1, using 4-hydroxybenzaldehyde (0.65 mmol, 80 mg) and (methoxycarbonylmethyl)triphenylphosphine (0.98 mmol, 328 mg) in water (4.0 mL) at 90°C for 1 h. The crude product was purified using flash chromatography (EtOAc/ hexane, 10:1 → 3:1) to give 91% (106 mg) of **8** as a white solid. m.p. 132-134°C (Reference m.p. 128-133°C [J. Org. Chem., 72, (2007), pp. 5244-5259]). $^1$H NMR (500 MHz, $CDCl_3$) δ 7.66 (d, 1H, $CH^{\beta}$=$CH^{\alpha}$, J = 16 Hz), 7.43-7.40 (m, aromatic, 2H), 6.87-6.85 (m, aromatic, 2H), 6.28 (d, 1H, $CH^{\beta}$=$CH^{\alpha}$, J = 16 Hz), 6.03 (br s, *H*OAr, 1H), 3.8 (s, $CH_3$, 3H); $^{13}$C NMR (125 MHz, $CDCl_3$) δ 168.3, 157.9, 144.9,130.0, 126.9, 115.9,114.9, 51.7; HR-MS (ES-) calc. for $C_{10}H_9O_3$ $(M)^-$: 177.0552. Found: 177.0552.

**Example 4**

**3-(2',4'-dihydroxyphenyl)-(*E*)-propenoic acid methyl ester (11)**

11 was obtained according to the procedure described in Example 1, using 2,4-dihydroxybenzaldehyde (0.72 mmol, 100 mg) and (methoxycarbonylmethyl)triphenylphosphine (1.08 mmol, 363 mg) in water (5.0 mL) at 90°C for 5 h. The crude product was purified using flash chromatography (EtOAc/ hexane, 1:1) to give 86% (120 mg) of **11** as a white solid. m.p. 158-160 °C. $^1$H NMR (500 MHz, $CDCl_3$+$CD_3OD$) δ 7.93 (d, 1H, $CH^{\beta}$=$CH^{\alpha}$, J = 16 Hz), 7.31 (d, aromatic, 1H, J = 8.5 Hz), 6.44 (d, 1H, $CH^{\beta}$=$CH^{\alpha}$, J = 16 Hz), 6.35 (dd, aromatic, 1H, J = 2 Hz, J = 8.5 Hz), 6.31 (d, aromatic, 1H, J = 2 Hz), 3.77 (s, $CH_3$, 3H); $^{13}$C NMR (125 MHz, $CDCl_3$+$CD_3OD$) δ 169.2, 160.1, 158.1, 141.2, 130.4, 113.8, 113.5, 107.7, 102.4, 51.2; HR-MS (ES-) calc. for $C_{10}H_9O_4$ $(M)^-$: 193.0501. Found: 193.0503.

**Example 5**

**3-(2',3',4'-trihydroxyphenyl)-(*E*)-propenoic acid methyl ester (12)**

**12** was obtained according to the procedure described in Example 1, using 2,3,4-trihydroxybenzaldehyde (12.1 mmol, 1.86 g) and (methoxycarbonylmethyl)triphenylphosphine (15.73 mmol, 5.26 g) in water (65 mL) at 90°C for 1 h. The crude product was purified using flash chromatography (chloroform/methanol, 10:1 → 1:1) to give 74% (1.85 g) of **12** as a bright brown solid. m.p. 149-153 °C.$^1$H NMR (500 MHz, $CDCl_3$) δ 7.87 (d, 1H, $CH^{\beta}$=$CH^{\alpha}$, J = 16.1 Hz), 6.88 (d, aromatic, 1H, J = 8.5 Hz), 6.43 (d, 1H, $CH^{\beta}$=$CH^{\alpha}$, J = 16.1 Hz), 6.35 (d, aromatic, 1H, J = 8.5 Hz), 3.74 (s, $CH_3$, 3H);

$^{13}C$ NMR (125 MHz, $CDCl_3$) δ 170.6, 149.6, 148.2, 143.1, 134.0, 121.2, 115.4, 114.5, 108.5, 51.8; HR-MS (ES-) calc. for $C_{10}H_9O_5$ $(M)^-$: 209.0450. Found: 209.0446.

**Example 6**

**3-(2',4',5'-trihydroxyphenyl)-(*E*)-propenoic acid methyl ester (13)**

13 was obtained according to the procedure described in Example 1, using 2,4,5-trihydroxybenzaldehyde (1.29 mmol, 200 mg) and (methoxycarbonylmethyl)triphenylphosphine (1.68 mmol, 560 mg) in water (7 mL) at 90°C for 30 min. The crude product was purified using flash chromatography (chloroform/methanol, 10:1 → 1:1) to give 37% (100 mg) of 13 as a bright brown solid. m.p. 150-152°C. $^1H$ NMR (600 MHz, $CD_3OD$) δ 7.91 (d, 1H, $CH^\beta$=$CH^\alpha$, J = 16.2 Hz), 6.89 (s, aromatic, 1H), 6.34 (s, aromatic, 1H), 6.28 (d, 1H, $CH^\beta$=$CH^\alpha$, J = 16.2 Hz), 3.74 (s, CH3,3H); $^{13}C$ NMR (150 MHz, $CD_3OD$) δ 170.6, 152.9, 150.8, 142.4, 139.8, 114.6, 113.7, 113.4, 104.2, 51.8; HR-MS (ES-) calc. for $C_{10}H_9O_5$ $(M)^-$: 209.0450. Found: 209.0447.

**Example 7**

**3-(4'-hydroxy-3-metoxyphenyl)-(*E*)-propenoic acid methyl ester (14)**

14 was obtained according to the procedure described in Example 1, using 4-hydroxy-3-methoxybenzaldehyde (0.65 mmol, 100 mg) and (methoxycarbonylmethyl)triphenylphosphine (0.98 mmol, 329 mg) in water (4 mL) at 90°C for 0.5 h. The crude product was purified using flash chromatography (EtOAc/ hexane, 5:1→1:1) to give 99% (134 mg) of 14 as a white solid. m.p. 67-69 °C (Reference m.p. 62-63°C [Arch. Pharm. Res., 28, 5, (2005), pp. 518-528]). $^1H$ NMR (500 MHz, $CDCl_3$) δ 7.63 (d, 1H, $CH^\beta$=$CH^\alpha$, J = 16 Hz), 7.08 (d, aromatic, 1H, J = 1.5 Hz), 7.07 (m, aromatic, 1H, J = 1.5 Hz, J = 2 Hz), 7.02 (m, aromatic, 1H, J = 1.5 Hz), 6.27 (d, 1H, $CH^\beta$=$CH^\alpha$, J = 16 Hz), 3.92 (s, $CH_3$, 3H), 3.79 (s, $CH_3$, 3H); $^{13}C$ NMR (125 MHz, $CDCl_3$) δ 167.7, 147.9, 146.7, 144.9, 126.9, 123.0, 115.1, 114.7, 109.3, 55.9, 51.5; HR-MS (ES-) calc. for $C_{11}H_{12}O_4$ $(M)^-$: 207.0657. Found: 207.0654.

**Example 8 (comparative)**

**4-(2'-hydroxyphenyl)-3(*E*)-buten-2-one (6)**

**6** was obtained according to the procedure described in Example 1, using 2-hydroxybenzaldehyde (0.82 mmol, 85 μL) and 1-(triphenylphosphoranylidene)-2-propanone (1.23 mmol, 391 mg) in water (4.0 mL) at 90°C for 2.5 h. The crude product was purified using flash chromatography (EtOAc/ hexane, 5:1 → 1:1) to give 85% (112 mg) of 6 as a yellow solid. m.p. 137-139 °C, (reference m.p. 139-140°C [Eur. J. Org. Chem., (2008), pp. 2759-2766]). $^1H$ NMR (500 MHz, $CDCl_3$) δ 7.78 (d, 1H, $CH^\beta$=$CH^\alpha$, J = 16.5 Hz), 7.46 (dd, aromatic, 1H, J = 1 Hz, J = 8 Hz), 7.27 (dd, aromatic, 1H, J = 1.5 Hz, J = 15.5 Hz), 7.05 (d, 1H, $CH^\beta$=$CH^\alpha$, J = 16.5 Hz), 6.94-6.92 (m, aromatic, 2H), 2.43 (s, $CH_3$, 3H); $^{13}C$ NMR (125 MHz, $CDCl_3$) δ 201.1, 156.0, 140.7, 131.9, 129.7, 127.7, 121.5, 120.6, 116.5, 26.8; HR-MS (ES-) calc. for $C_{10}H_9O_2$ $(M)^-$: 161.0603. Found: 161.0604.

**Example 9 (comparative)**

**4-(3'-hydroxyphenyl)-3(*E*)-buten-2-one (15)**

15 was obtained according to the procedure described in Example 1, using 3-hydroxybenzaldehyde (0.82 mmol, 100 mg) and 1-(triphenylphosphoranylidene)-2-propanone (1.23 mmol, 391 mg) in water (4.0 mL) at 90°C for 3 h. The crude product was purified using flash chromatography (EtOAc/ hexane, 5:1 → 1:1) to give 97% (128 mg) of 15 as a white solid. m.p. 94-96°C, (reference m.p. 96-97°C [Environ. Chem. Lett, 14, (2016), pp. 223-228]). $^1H$ NMR (500 MHz, $CDCl_3$) δ 7.50 (d, 1H, $CH^\beta$=$CH^\alpha$, J = 16.0 Hz), 7.28-7.24 (m, aromatic, 1H), 7.10-7.08 (m, aromatic, 2H), 6.94-6.92 (m, aromatic, 1H), 6.71 (d, 1H, $CH^\beta$=$CH^\alpha$, J = 16.0 Hz), 2.40 (s, $CH_3$, 3H); $^{13}C$ NMR (125 MHz, $CDCl_3$) δ 199.7, 156.5, 144.2, 135.7, 130.2, 127.1, 121.0, 118.1,114.6,27.4; HR-MS (ES-) calc. for $C_{10}H_9O_2$ $(M)^-$: 161.0603. Found: 161.0602.

**Example 10 (comparative)**

**4-(4'-hydroxyphenyl)-3(*E*)-buten-2-one (16)**

16 was obtained according to the procedure described in Example 1, using 4-hydroxybenzaldehyde (0.82 mmol,

100 mg) and 1-(triphenylphosphoranylidene)-2-propanone (1.23 mmol, 391 mg) in water (4.0 mL) at 90°C for 2 h. The crude product was purified using flash chromatography (EtOAc/ hexane, 5:1 → 1:1) to give 83% (110 mg) of **16** as a white solid. m.p. 110-111°C, (reference m.p. 111-113°C [0rg. Process Res. Dev. 15, (2011), pp. 858-870]). $^{1}H$ NMR (500 MHz, $CDCl_3$) δ 7.53 (d, 1H, $CH^{\beta}=CH^{\alpha}$,J = 16.0 Hz), 7.45-7.43 (m, aromatic, 2H), 6.93-6.90 (m, aromatic, 2H), 6.62 (d, 1H, $CH^{\beta}=CH^{\alpha}$, J = 16.0 Hz), 2.39 (s, $CH_3$, 3H); $^{13}C$ NMR (125 MHz, $CDCl_3$) δ 200.1, 159.0, 144.8, 130.4, 126.4, 124.3, 116.2, 27.2; HR-MS (ES-) calc. for $C_{10}H_9O_2$ $(M)^-$: 161.0603. Found: 161.0609.

**Example 11**

**4-(2',4'-dihydroxyphenyl)-3(*E*)-buten-2-one (17)**

**17** was obtained according to the procedure described in Example 1, using 2,4-dihydroxybenzaldehyde (1.44 mmol, 200 mg) and 1-(triphenylphosphoranylidene)-2-propanone (1.88 mmol, 600 mg) in water (10.0 mL) at 90°C for 2 h. The crude product was purified using flash chromatography (EtOAc/ hexane, 5:1 → 1:1) to give 78% (200 mg) of 17 as a bright brown solid; m.p. 118-120°C decomp., $^{1}H$ NMR (500 MHz, $CD_3OD$) δ 7.91 (d, 1H, $CH^{\beta}=CH^{\alpha}$, J = 16.0 Hz), 7.39 (d, aromatic, 1H, J = 8 Hz), 6.71 (d, 1H, $CH^{\beta}=CH^{\alpha}$, J = 16.0 Hz), 6.34-6.32 (m, aromatic, 2H), 2.32 (s, $CH_3$, 3H); $^{13}C$ NMR (125 MHz, $CD_3OD$) δ 202.2, 162.9, 160.4, 142.6, 131.4, 124.1, 114.6, 109.1, 103.4, 26.7; HR-MS (ES-) calc. for $C_{10}H_9O_3$ $(M)^-$: 177.0552. Found: 177.0555.

**Example 12**

**4-(2',3',4'-trihydroxyphenyl)-3(*E*)-buten-2-one (18)**

18 was obtained according to the procedure described in Example 1, using 2,3,4-trihydroxybenzaldehyde (1.29 mmol, 200 mg) and 1-(triphenylphosphoranylidene)-2-propanone (1.94 mmol, 620 mg) in water (10.0 mL) at 90°C for 0.5 h. The crude product was purified using flash chromatography (EtOAc/ hexane, 2:1 → 1:1) to give 70 % (175 mg) of **18** as a bright brown solid; m.p. 98-100°C decomp. $^{1}H$ NMR (500 MHz, $CD_3OD$) δ 7.88 (d, 1H, $CH^{\beta}=CH^{\alpha}$, J = 16.1 Hz), 6.96 (d, aromatic, 1H, J = 8.7 Hz), 6.72 (d, 1H, $CH^{\beta}=CH^{\alpha}$, J = 16.1 Hz), 6.37 (d, aromatic,1H, J = 8.6 Hz), 2.33 (s, $CH_3$, 3H); $^{13}C$ NMR (125 MHz, $CD_3OD$) δ 202.1, 150.2, 148.5, 142.9, 134.0, 124.5, 121.1, 115.3, 108.7, 26.7; HR-MS (ES-) calc. for $C_{10}H_9O_4$ $(M)^-$: 193.0501. Found: 193.0506.

**Example 13**

**4-(3'-methoxy-4'-hydroxyphenyl)-3(*E*)-buten-2-one (20)**

20 was obtained according to the procedure described in Example 1, using 4-hydroxy-3-methoxybenzaldehyde (0.65 mmol, 100 mg) and 1-(triphenylphosphoranylidene)-2-propanone (0.98 mmol, 313 mg) in water (4.0 mL) at 90°C for 0.5 h. The crude product was purified using flash chromatography (EtOAc/ hexane, 5:1 → 2:1) to give 98% (124 mg) of 20 as a white solid; m.p. 126-128°C, (reference m.p. 126°C [Arch. Pharm. Res. 29, (2006), pp. 469-475]) $^{1}H$ NMR (500 MHz, $CDCl_3$) δ 7.46 (d, 1H, $CH^{\beta}=CH^{\alpha}$, J = 16.0 Hz), 7.10-7.07 (m, aromatic, 1H), 7.05 (d, aromatic, 1H, J = 1.5 Hz), 6.92 (d, aromatic, 1H, J = 8 Hz), 6.57 (d, 1H, $CH^{\beta}=CH^{\alpha}$, J = 16.0 Hz), 6.03 (br s, O*H,* 1H), 3.93 (s, $CH_3$, 3H), 2.36 (s, $CH_3$, 3H); $^{13}C$ NMR (125 MHz, $CDCl_3$) δ 198.4, 148.2, 146.9, 143.7, 126.8, 124.9, 123.5, 114.8, 109.3,55.9,27.2; HR-MS (ES-) calc. for $C_{11}H_{11}O_3$ $(M)^-$: 191.0708. Found: 191.0709.

**Example 14**

**Biological activity**

**Calculation of log $P_{o/w}$**

The partition coefficient between *n*-octanol and water (log $P_{o/w}$) was calculated using a SwissADME web tool (Daina, A. et al. SwissADME: a free web tool to evaluate pharmacokinetics, drug-likeness and medicinal chemistry friendliness of small molecules. Sci. Rep. 7,42717; doi: 10.1038/srep42717 (2017).

**Determination of the oxidation inhibition of the o/w emulsion**

The emulsion was prepared according to the method described in Zarnowski R. et al., The oil of Adenanthera pavonina L. seeds and its emulsions. Z Naturforsch C. 2004;59(5-6):321-326; Jaromin A. et al., Emulsions of oil from

Adenanthera pavonina L. seeds and their protective effect Cell Mol Biol Lett 2006; 11(3): 438-448). Briefly, 15 mg of sorbitan sesquioleate was added to 50 mg of flaxseed oil and vigorously mixed for 20 min. Then, 2 ml of an aqueous phase consisting of 10 mM Tris-HCl buffer, pH 7.4, was added. The sample was further vortexed for 15 min. and sonicated (Sonics & Materials Inc., Newton, CT, USA) for 10 min. in a coldwater bath. Then, 20 μl of the 1:1 (v/v) buffer diluted emulsion, methanolic solution of each compound (final concentration 100 μM) and 10 mM Tris-HCl buffer, pH 7.4, were mixed and incubated at room temperature for 10 min. To induce oxidation, 100 μl of freshly prepared aqueous solution of ammonium iron(II) sulfate hexahydrate (Mohr's salt) (5 mM) was added (total volume of the sample was 1 ml) and the samples were incubated at room temperature for 15 min. Flaxseed oil emulsion without any compound addition was included as control. After the oxidation step, determination of the thiobarbituric acid reacting substances (TBARS) was performed (Buege J.A., Aust S.D., Microsomal lipid peroxidation. Methods Enzymol. 1978;52:302-310). 2 ml of the thiobarbituric acid (TBA) reagent (15% trichloroacetic acid, 0.25 N HCl and 0.5% TBA) was added to each sample, mixed and heated at 100°C for 30 min. After centrifugation at 13 000 rpm for 10 min, the absorbance of the supernatants was measured at 535 nm. The obtained values were used to calculate the % of the oxidation inhibition according to the following equation:

$$\text{Inhibition of the oxidation (\%)} = [1-(\text{absorbance of the sample}/\text{absorbance of control})] \times 100$$

**Free radical scavenging activity**

The 2,2-diphenyl-1-picrylhydrazyl (DPPH) scavenging assay was carried out according to the procedure described in Zhang Z1. et al., Synthesis and biological evaluation of caffeic acid 3,4-dihydroxyphenethyl ester. J Nat Prod. 2010 Feb 26; 73(2): 252-4. doi: 10.1021/np900519d.) with a modification. Briefly, different concentrations of the compounds (100 μl) were mixed with 900 μl of the 0.04 mg/ml methanolic solution of DPPH. The mixture was kept at room temperature for 30 min, and then the absorbance at 517 nm was measured. The half maximal inhibitory concentration ($IC_{50}$) was used as an indicating measure of the scavenging activity.

The antioxidative activity test results for the compounds prepared by the process of the invention are presented in Table 2 below.

Table 2. Log $P_{o/w}$ and antioxidative activity of the reference and tested compounds.

| **Compound** | **Log $P_{o/w}$** | **o/w emulsion[a]** | **DPPH $IC_{50}$ [μM]** |
|---|---|---|---|
| **CA** | 0.93 | 26.1 ± 2.9 | 32.2 |
| **9*** | 1.82 | 2.6 ± 1.5 | *n.a.*[c] |
| **10*** | 1.78 | 18.4 ± 2.2 | *n.a.*[c] |
| **8*** | 1.81 | 22.7 ± 6.4 | *n.a.*[c] |
| **11** | 1.31 | **33.7 ± 6.5** | **1015.9** |
| **12** | 0.99 | **55.5 ± 2** | **18.6** |
| **13** | 0.95 | **38.6 ± 2.3** | **53.3** |
| **14** | 1.76 | 14.8 ± 2 | 58.9 |
| **6*** | 1.94 | *n.a.*[b] | *n.a.*[c] |
| **15*** | 1.82 | *n.a.*[b] | *n.a*[c] |
| **16*** | 1.85 | 6.8 ± 4.4 | *n.a.*[c] |
| **17** | 1.49 | 4.6 ± 1.3 | 688.2 |
| **18** | 1.05 | **28.6 ± 4** | **16.8** |

(continued)

| Compound | Log $P_{o/w}$ | o/w emulsion[a] | DPPH $IC_{50}$ [μM] |
|---|---|---|---|
| **20** | 1.86 | 7.9 ± 2.1 | 61.8 |
| * reference compound<br>[a] determined at 100 μM.<br>[b] no protection detected.<br>[c] $IC_{50}$ > 5 mM. | | | |

**Cell viability assay in normal human dermal fibroblast (NHDF) cell line**

The NHDF cell line was cultured in the Minimum Essential Medium Eagle Alpha Modifications medium supplemented with a 10% fetal bovine serum and 2 mM glutamine,100 U/ml penicillin,100 μg/ml streptomycin, and 25 μg/ml amphotericin B. The cells were cultured in a 37°C incubator in humidified atmosphere containing 5% of $CO_2$. Cell viability was determined by the MTT method as described earlier (Mosmann T., Rapid colorimetric assay for cellular growth and survival: application to proliferation and cytotoxicity assays. J Immunol Methods. 1983;65(1-2):55-63). NHDF cells were seeded in triplicate into 96-well culture plates at $10^4$ cells/well and incubated at 37°C in a humidified atmosphere and 5% of $CO_2$ overnight. Cells, in triplicate wells, were treated with the methanolic solution of the tested compounds to obtain the final concentration of 12.5, 25, 50, 100, 200 or 250 μM, and then incubated for 24 and 48 hours. Subsequently, the medium was carefully removed from the wells, followed by the addition of 50 μl of the dye solution (0.5 mg/ml of MTT salt in the culture medium) and incubated at 37°C for 4 hours. After incubation the medium was removed and 50 μl of DMSO was added to dissolve the formazan crystals. The absorbance of the samples was measured at 550 nm with the reference wavelength of 630 nm on a microplate reader UVM 340 (Biogenet, Poland). The untreated control was normalized to 100% for each assay, and treatments were expressed as the percentage of control (cell exposed to the highest tested volume of methanol). Viability of NHDF incubated with the compounds obtained by the process of the invention after a 24h (A) and 48h (B) exposure to different concentrations of CA and its derivatives is presented in Fig. 1. All compounds were tested at the concentrations of 12.5-250 μM.

## Claims

1. A process for the preparation of the caffeic acid derivatives substituted with at least two hydroxyl groups in the aromatic ring, and represented by formula (I)

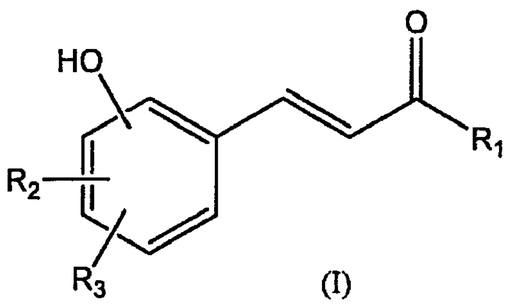

(I)

wherein

$R_1$ is $-CH_3$ or $-OCH_3$,
$R_2$ is OH or $-OCH_3$, and
$R_3$ is H or OH,

in the reaction of an aromatic aldehyde with the unprotected hydroxyl groups represented by formula (II)

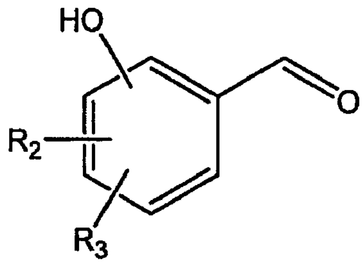

(II)

and ylide of formula (III)

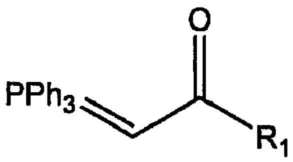

(III)

wherein $R_1$, $R_2$ and $R_3$ have the meaning defined for formula (I), under the Horner-Wadsworth-Emmons reaction conditions, wherein the reaction is carried out in water.

**2.** The process according to Claim 1, wherein the reaction is carried out at 70-100°C.

**3.** The process according to Claims 1-2, wherein the reaction is carried out at 90°C.

**4.** The process according to Claim 1, wherein the said process is used for the preparation of the compounds of formula (I) substituted with two OH groups, wherein $R_1$ is $-OCH_3$ or $-CH_3$, OH is in the position 2 of the aromatic ring, $R_2$ is OH, and $R_3$ is H.

**5.** The process according to Claim 1, wherein the said process is used for the preparation of the compounds of formula (I) substituted with two OH groups, wherein $R_1$ is $-OCH_3$ or $-CH_3$, $R_2$ is $-OCH_3$ in the position 2, and $R_3$ is OH in the position 3 of the aromatic ring.

**6.** The process according to Claim 1, wherein the said process is used for the preparation of the compounds of formula (I) substituted with three OH groups, wherein $R_1$ is $-OCH_3$ or $-CH_3$, and both $R_2$ and $R_3$ are OH, under the provision that when $R_1$ is $-OCH_3$, neither $R_2$ nor $R_3$ is in the position 5 of the aromatic ring.

**7.** The compounds of formula (I) selected from the group comprising:

3-(2',4',5'-trihydroxyphenyl)-(*E*)-propenoic acid methyl ester, and
4-(2',3',4'-trihydroxyphenyl)-3(*E*)-buten-2-one.

**8.** The use of the caffeic acid derivatives prepared by the process according to any of Claims 1 - 6 as the antioxidants in pharmaceuticals and cosmetics.

**9.** The use of the caffeic acid derivatives prepared by the process according to any of Claims 1 - 6 as the active agents for skin or hair care products.

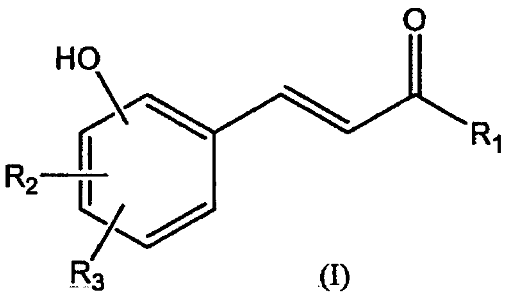

(I)

A

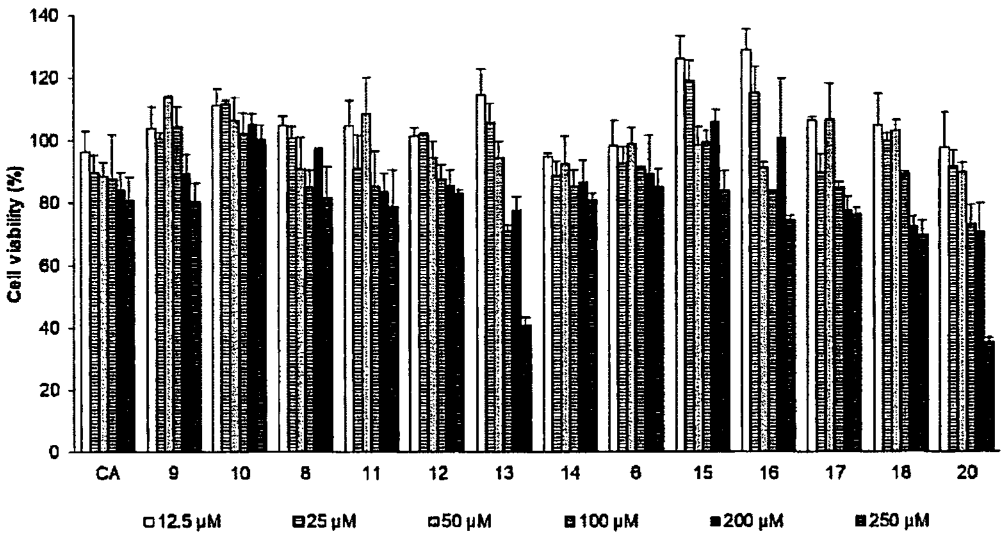


B

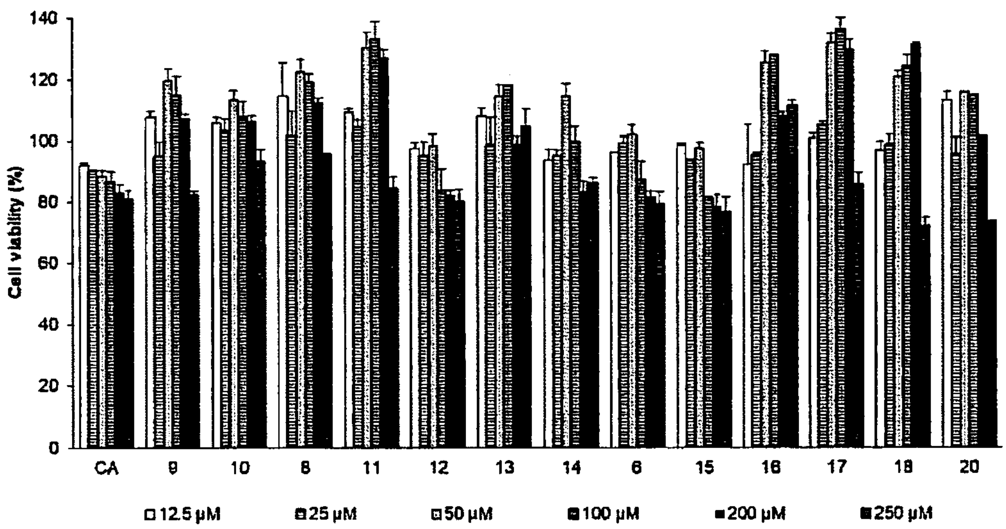


**Fig. 1.** Cell viability in normal human dermal fibroblast (NHDF) cells after a 24h (A) and 48h (B) exposure to different concentrations of CA and its derivatives. The cell viability was evaluated with the MTT assay and the results were presented as the percentage of the control groups. Data are presented as the mean standard deviation (SD) of three independent experiments.

Europäisches Patentamt
European Patent Office
Office européen des brevets

# EUROPEAN SEARCH REPORT

Application Number
EP 18 00 0565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZOLTÁN PÜNKÖSTI ET AL: "Synthesis of 7-Azido-3-Formylcoumarin - A Key Precursor in Bioorthogonally Applicable Fluorogenic Dye Synthesis: Bioorthogonal Fluorogenic Dyes", JOURNAL OF HETEROCYCLIC CHEMISTRY, vol. 55, no. 5, 12 March 2018 (2018-03-12), pages 1183-1188, XP055529212, US ISSN: 0022-152X, DOI: 10.1002/jhet.3151 * Preparation of compound 2f in Scheme 1 * | 1-4 | INV. C07C67/343 C07C69/732 C07C45/72 C07C49/248 A61K8/35 A61K8/37 A61K31/12 A61K31/216 A61Q17/04 A61P17/18 |
| Y | BRAVO PIERFRANCESCO ET AL.: "The Reaction of Carbonyl-Stabilized Phosphonium and Arsonium Ylids with o-Hydroxy Benzaldehydes. Synthesis of o-Hydroxy Chalcones", GAZZETTA CHIMICA ITALIANA, vol. 105, 1 January 1975 (1975-01-01), pages 109-115, XP009509797, * Preparation of compound VIIIh in Table 1 * | 1-4 | |
| | | | TECHNICAL FIELDS SEARCHED (IPC) |
| Y,D | DAMBACHER J ET AL: "Water is an efficient medium for Wittig reactions employing stabilized ylides and aldehydes", TETRAHEDRON LETTERS, ELSEVIER, AMSTERDAM, NL, vol. 46, no. 26, 27 June 2005 (2005-06-27), pages 4473-4477, XP027863885, ISSN: 0040-4039 [retrieved on 2005-06-27] * the whole document * * table 1 * * page 4475, left-hand column, paragraph 3 * | 1-6 | C07C A61K A61P A61Q |

-/--

1 ~~The present search report has been drawn up for all claims~~

| Place of search | Date of completion of the search | Examiner |
|---|---|---|
| The Hague | 7 December 2018 | Mooren, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 00 0565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHIROU MAEDA ET AL: "Studies on the Preparation of Bioactive Lignans by Oxidative Coupling Reaction. IV. Oxidative Coupling Reaction of Methyl (E)-3-(3, 4-Dihydroxy-2-methoxyphenyl)propenoate and Lipid Peroxidation Inhibitory Effects of the Produced Lignans", CHEM. PHARM. BULL., vol. 43, no. 1, 15 January 1995 (1995-01-15), pages 84-90, XP055530100, * Preparation of compound 11 in Chart 4 * ----- | 1-3,5 | |
| Y | SOMEPALLI VENKATESWARLU ET AL: "Antioxidant and Antimicrobial Activity Evaluation of Polyhydroxycinnamic Acid Ester Derivatives.", CHEMINFORM, vol. 37, no. 18, 1 January 2006 (2006-01-01), pages 252-257, XP055530122, DE ISSN: 0931-7597, DOI: 10.1002/chin.200618070 * Preparation of compound 5m in Scheme 1 * ----- | 1-3,6 | TECHNICAL FIELDS SEARCHED (IPC) |
| T | KATARZYNA SIDORYK ET AL: "Synthesis and Antioxidant Activity of Caffeic Acid Derivatives", MOLECULES ONLINE, vol. 23, no. 9, 30 August 2018 (2018-08-30), page 2199, XP055529208, DE ISSN: 1433-1373, DOI: 10.3390/molecules23092199 ----- | | |

1

~~The present search report has been drawn up for all claims~~

| Place of search | Date of completion of the search | Examiner |
|---|---|---|
| The Hague | 7 December 2018 | Mooren, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches Patentamt
European Patent Office
Office européen des brevets

Application Number
EP 18 00 0565

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 18 00 0565

The Search Division considers that the present European patent application does not comply with the requirements of unity of invention and relates to several inventions or groups of inventions, namely:

1. claims: 1-6

   Processes for the preparation of caffeic acid derivatives of formula (I)

   ---

2. claims: 7-9

   Compounds of formula (I); the uses of compounds prepared by a process according to claim 1 as antioxidants in pharmaceuticals and cosmetics or as active agents for skin or hair products

   ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **P. ZHANG et al.** Bioactivity and chemical synthesis of caffeic acid phenethyl ester and its derivatives. *Molecules,* 2014, vol. 19, 16458-16476
- **S. QUIDEAU et al.** Plant polyphenols: chemical properties, biological activities, and synthesis. *Angew. Chem. Int Ed.,* 2011, vol. 50, 586-621
- **C.A. GOMES et al.** Anticancer activity of phenolic acids of natural or synthetic origin: a structure-activity study. *J. Med. Chem.,* 2003, vol. 46, 5395-5401
- **H.M. EID et al.** Structural constraints and the importance of lipophilicity for the mitochondrial uncoupling activity of naturally occurring caffeic acid esters with potential for the treatment of insulin resistance. *Biochem. Pharmacol.,* 2010, vol. 79, 444-454
- **K. TAKAHASHI et al.** Methyl caffeate as an α-glucosidase inhibitor from Solanum torvum fruits and the activity of related compounds. *Biosci. Biotechnol. Biochem.,* 2010, vol. 74 (4), 741-745
- **J. GANUGAPATI et al.** Molecular docking studies of antidiabetic activity of cinnamon compounds. *Asian J. of Pharm. and Clinical Res.,* 2014, vol. 7, 2
- **S. M. FIUZA et al.** Phenolic acid derivatives with potential anticancer properties - a structure-activity relationship study. Part 1: Methyl, propyl and octyl esters of caffeic and gallic acids. *Bioorg. Med. Chem.,* 2004, vol. 12, 3581-3589
- **L.S. MONTEIRO et al.** Synthesis and preliminary biological evaluation of new phenolic and catecholic dehydroamino acid derivatives. *Tetrahedron.,* 2017, vol. 73, 6199-6209
- **J.H. CHEN ; H. CHI-TANG.** Antioxidant activities of caffeic acid and its related hydroxycinnamic acid compounds. *J. Agric. Food Chem.,* 1997, vol. 7 (45), 2374-2378
- **Y. SATO et al.** In vitro and in vivo antioxidant properties of chlorogenic acid and caffeic acid. *Int J. Pharm.,* 2010, vol. 1-2 (403), 136-138
- **S. LOSADA-BARREIRO ; C. BRAVO-DFAZ.** Free radicals and polyphenols: The redox chemistry of neurodegenerative diseases. *Eur. J. Med. Chem.,* 2017, vol. 133, 379-402
- **V. SRIVASTAVA et al.** Synthesis of diverse analogues of Oenostacin and their antibacterial activities. *Bioorg. Med. Chem.,* 2007, vol. 15, 518-525
- **CH. SIQUET et al.** Antioxidant profile of dihydroxy- and trihydroxyphenolic acids - A structure-activity relationship study. *Free Radical Res.,* 2006, vol. 40 (4), 433-442
- **P.C. KUO et al.** Isolation of a natural antioxidant, dehydrozingerone from Zingiber officinale and synthesis of its analogues for recognition of effective antioxidant and antityrosinase agents. *Arch. Pharm. Res.,* 2005, vol. 28 (5), 518-528
- **I.E. ALVARADO et al.** Fungal biotransformation of p-coumaric acid into caffeic acid by Pycnoporus cinnabarinus: an alternative for producing a strong natural oxidant. *World J., Microbiol. Biotechnol.,* 2003, vol. 19, 157-160
- **P.A KROON ; G. WILLIAMSON.** Hydroxycinnamates in plants and food: current and future perspectives. *J. Sci. Food Agric.,* 1999, vol. 79, 355-361
- **M. TOUAIBIA et al.** Caffeic acid, a versatile pharmacophore: an overview. *Mini Rev. Med. Chem.,* 2011, vol. 11, 695-713
- **T. FURUYA et al.** Biotechnological production of caffeic acid by bacterial cytochrome P450 CYP199A2. *Appl. Environ Microbiol.,* 2012, vol. 78 (17), 6087-6094
- **A. JAFARI ; M. GHADAMI.** Efficient synthesis of α,β-unsaturated ketones with trans-selective Horner-Wadsworth-Emmons reaction in water. *Environ. Chem. Lett.,* 2016, vol. 14, 223-228
- **B. LIST.** *Angew. Chem. Int. Ed.,* 2010, vol. 49, 1730-1734
- **G. GUILLENA ; C. NAJERA ; D. RAMON.** Enantioselective direct aldol reaction: the blossoming of modern organocatalysis. *Tetrahedron: Asymmetry,* 2007, vol. 18, 2249-2293
- **N. SAITO et al.** Guanidine-catalyzed asymmetric synthesis of 2,2-disubstituted chromane skeletons by intramolecular oxa-Michael addition. *Eur. J. Org. Chem.,* 2008, 2759-2766
- **V.J. PATIL ; U. MAVERS.** Wittig reactions in the presence of Silica gel. *Tetrahedron Lett,* 1996, vol. 37, 1281-1284
- **N.S. ISAACS ; G.N. EL-DIN.** The application of high pressure to some difficult Wittig reactions. *Tetrahedron Lett.,* 1987, vol. 28, 2191-2192
- **D.L. HOOPER ; S. GARAGAN ; M.M. KAYSER.** Lithium cation-catalyzed Wittig reactions. *J. Org. Chem.,* 1994, vol. 59, 1126-1128

- **V.L. BOULAIRE ; R. GREE.** Wittig reactions in the ionic solvent [bmim][BF. *Chem. Commun.,* 2000, 2195-2196
- **A. SPINELLA et al.** Microwave accelerated Wittig reactions of stabilized phosphorus ylides with ketones under solvent-free conditions. *Synlett,* 1997, 93-94
- **A.A. JAFARI ; M. GHADAMI.** Efficient synthesis of α,β-unsaturated ketones with trans-selective Horner-Wadsworth-Emmons reaction in water. *Environ. Chem. Lett.,* 2016, vol. 14, 223-228
- **J. DAMBACHER et al.** Water is an efficient medium for Wittig reactions employing stabilized ylides and aldehydes. *Tetrahedron Lett,* 2005, vol. 46, 4473-4477
- **A. EL-BATTA et al.** Wittig reactions in water media employing stabilized ylides with aldehydes. Synthesis of α,β-unsaturated esters from mixing aldehydes, α-bromoesters, and Ph3P in aqueous NaHCO. *J. Org. Chem.,* 2007, vol. 72, 5244-5259
- **J. DAMBACHER et al.** Water is an efficient medium for Wittig reactions employing stabilized ylides and aldehydes. *Tetrahedron Lett.,* 2005, vol. 46, 4473-4477
- **A. NONNENMACHER et al.** Application of highpressure on Wittig reactions with resonance stabilized ylides. *Liebigs Ann. Chem.,* 1983, vol. 12, 2135-2140
- **A. SAIJA et al.** Ferulic and caffeic acids as potential protective agents against photooxidative skin damage. *J. Sci. Food Agric.,* 1999, vol. 79, 476-480
- **SAIJA, A. et al.** In vitro and in vivo evaluation of caffeic and ferulic acids as topical photoprotective agents. *Int. J. Pharm.,* 2000, vol. 199, 39-47
- **PLUEMSAMRAN, T. et al.** Caffeic acid and ferulic acid inhibit UVA-induced matrix metalloproteinase-1 through regulation of the antioxidant defense system in keratinocyte HaCaT cells. *Photochem. Photobiol.,* 2012, vol. 88, 961-968
- *Chem. Med. Chem.,* 2010, vol. 5, 598-607
- *J. Org. Chem.,* 2007, vol. 72, 5244-5259
- *Arch. Pharm. Res.,* 2005, vol. 28 (5), 518-528
- *Eur. J. Org. Chem.,* 2008, 2759-2766
- *Environ. Chem. Lett,* 2016, vol. 14, 223-228
- *0rg. Process Res. Dev.,* 2011, vol. 15, 858-870
- *Arch. Pharm. Res.,* 2006, vol. 29, 469-475
- **DAINA, A. et al.** SwissADME: a free web tool to evaluate pharmacokinetics, drug-likeness and medicinal chemistry friendliness of small molecules. *Sci. Rep.,* 2017, vol. 7, 42717
- **ZARNOWSKI R. et al.** The oil of Adenanthera pavonina L. seeds and its emulsions. *Z Naturforsch C.,* 2004, vol. 59 (5-6), 321-326
- **JAROMIN A. et al.** Emulsions of oil from Adenanthera pavonina L. seeds and their protective effect. *Cell Mol Biol Lett,* 2006, vol. 11 (3), 438-448
- **BUEGE J.A. ; AUST S.D.** Microsomal lipid peroxidation. *Methods Enzymol.,* 1978, vol. 52, 302-310
- **ZHANG Z1. et al.** Synthesis and biological evaluation of caffeic acid 3,4-dihydroxyphenethyl ester. *J Nat Prod.,* 26 February 2010, vol. 73 (2), 252-4
- **MOSMANN T.** Rapid colorimetric assay for cellular growth and survival: application to proliferation and cytotoxicity assays. *J Immunol Methods,* 1983, vol. 65 (1-2), 55-63